Europäisches Patentamt

European Patent Office

Office européen des brevets

(19)

(11) Publication number: **0 376 434**
**A2**

## (12) EUROPEAN PATENT APPLICATION

(21) Application number: **89307210.8**

(22) Date of filing: **17.07.89**

(51) Int. Cl.⁵: **G06K 9/46, G07C 9/00**

(30) Priority: **29.12.88 JP 330918/88**

(43) Date of publication of application:
**04.07.90 Bulletin 90/27**

(84) Designated Contracting States:
**DE FR GB**

(71) Applicant: **KABUSHIKI KAISHA TOSHIBA**
**72, Horikawa-cho Saiwai-ku**
**Kawasaki-shi Kanagawa-ken 210(JP)**

(72) Inventor: **Takeda, Masahiro c/o Patent**
**Division**
**Kabushiki Kaisha Toshiba 1-1 Shibaura**
**1-chome**
**Minato-ku Tokyo(JP)**
Inventor: **Uchida, Satoshi c/o Patent Division**
**Kabushiki Kaisha Toshiba 1-1 Shibaura**
**1-chome**
**Minato-ku Tokyo(JP)**

(74) Representative: **Sturt, Clifford Mark et al**
**MARKS & CLERK 57-60 Lincoln's Inn Fields**
**London WC2A 3LS(GB)**

(54) **Apparatus for registering individualities and for identifying individuals.**

(57) In an apparatus for registering individualities is equipped the same data representing physical features of an individual is input, a plurality of times, the physical features stored in distinctive feature storage units is compared with each other, and what should be registered from among the data thus input on the basis of the results of comparison is selected. More specifically, a plurality of signals are input at the time of registration and a combination of any given two signals is processed for comparison to determine the quality of each signal to be registered. One of the signals which has been selected as a good one is accordingly registered, so that registration of individualities is implemented for certain.

## APPARATUS FOR REGISTERING INDIVIDUALITIES AND FOR IDENTIFYING INDIVIDUALS

The present invention relates to an apparatus for registering individual physical features and an apparatus for identifying an individual.

There is an apparatus for identifying an individual using a signal gained by adding up finger-image densities in the direction perpendicular to the longitudinal direction of a finger. The method of identifying an individual by means of the apparatus therefor comprises putting in correct alignment the signal, which is gained by adding up finger-image densities derived from the difference between the contact and noncontact of the finger with a finger rest on which the finger is placed, and an added-up signal preregistered in a dictionary, and comparing the former with the latter.

This registering process is conducted by computing the square errors of these two added-up signals while one of the signals is being shifted.

In the case of such an apparatus for identifying an individual, however, the disadvantage is that identification of the individual will end up in failure if the input itself is bad at the time of registration. In addition to that disadvantage, unsatisfactory identifying accuracy is problematical.

In view of the foregoing, an object of the present invention is to provide an apparatus for having individualities registered for certain and an apparatus for identifying an individual with accuracy.

In order to accomplish the aforesaid object, an apparatus for registering individualities according to the present invention comprises input means for inputting the same data representing physical features of an individual a plurality of times, comparison means for comparing the data representing the physical features thus input by the input means, and selection means for selecting what should be registered from among the data thus input on the basis of the results of comparison made by the comparison means.

Fig. 1 is a block diagram illustrating the configuration of an apparatus for registering individualities.

Fig. 2 is a flowchart showing the operation of the apparatus for registering individualities.

Fig. 3 is a diagram showing direction codes.

Fig. 4 is a block diagram illustrating the configuration of an apparatus for identifying an individual.

Fig. 5 is a diagram showing a method of placing a finger in the apparatus for identifying an individual.

Figs. 6 and 7 are flowcharts showing the operation of the apparatus for identifying an individual.

Referring to the accompanying drawings, a detailed description will subsequently be given of the preferred embodiment of the present invention.

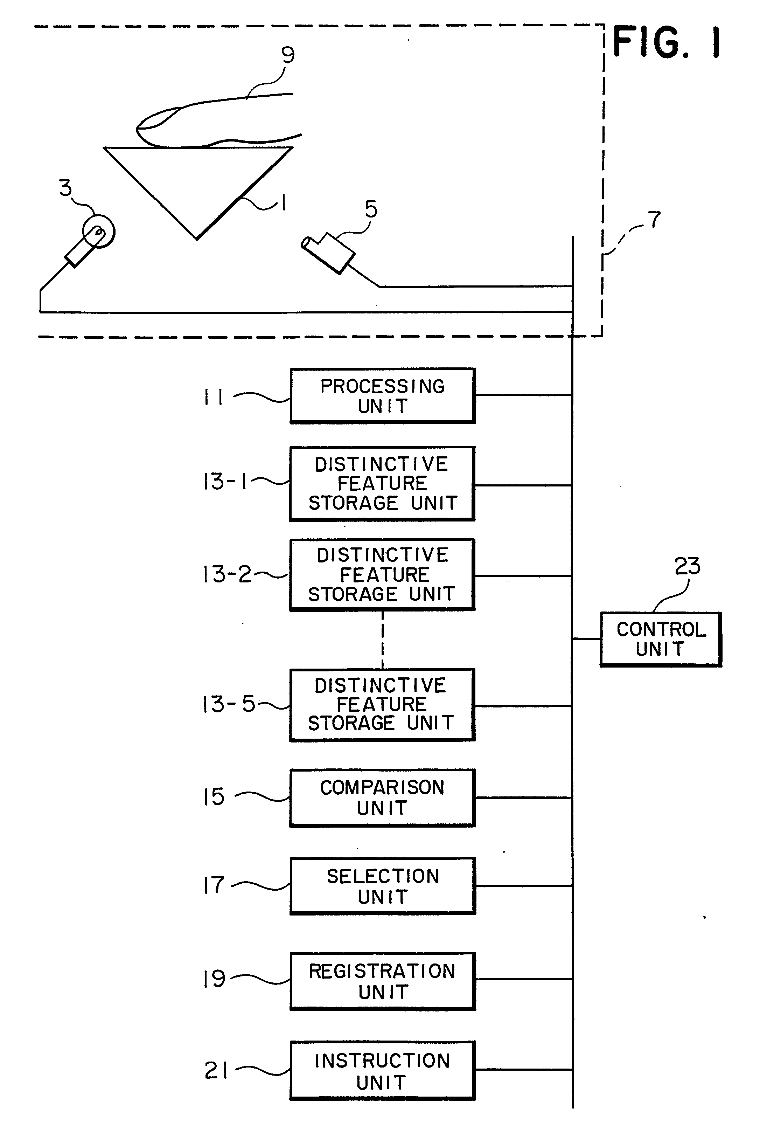

Fig. 1 shows an embodiment of an apparatus for registering individualities. The apparatus for registering individualities is designed to select the distinctive features of a fingerprint in the best condition from among those input five times when the fingerprint is registered.

As shown in Fig. 1, the apparatus for registering individualities comprises a right-angled equilateral prism 1, an illuminating means 3, a read means 7 having a television camera 5, a processing unit 11, distinctive feature storage units 13-1,...13-5, a comparison unit 15, a selection unit 17, a registration unit 19, an instruction unit 21, and a control unit 23. A finger 9 is placed on the right-angled equilateral prism 1.

The illuminating means 3 irradiates the right-angled equilateral prism 1. The television camera 5 reads the light reflected from the finger 9 via the right-angled equilateral prism 1. The processing unit 11 converts the signal sent from the television camera 5 into a binary value, divides the whole finger image into 32 x 32 pieces of blocks, quantizes a representative direction within a fingerprint pattern present in each block into eight directions and extracts the directions thus quantized.

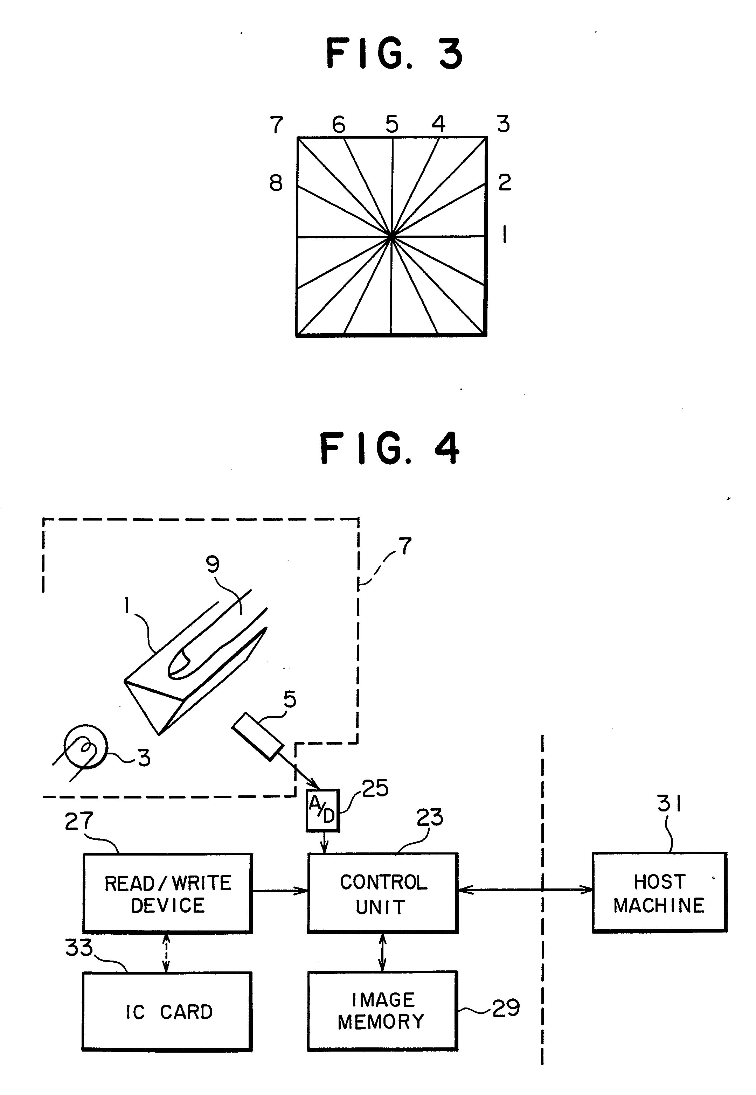

Fig. 3 shows the definition of the direction codes.

The distinctive feature storage units 13-1,...13-5 store the signals of the finger 9 read by the television camera 5. At this time, the television camera 5 reads the signal of the finger 9 five times, so that the signal read out once is stored in each of the distinctive feature storage units 13-1,...13-5. The comparison unit 15 compares the signals of the finger 9 stored in the distinctive feature storage units 13-1,...13-5.

The selection unit 17 selects one of the signals to be registered from among those stored in the distinctive feature storage units 13-1,...13-5 according to the results of comparison made by the comparison unit 15. The registration unit 19 selects the signal selected by the selection unit 17.

The instruction unit 21 instructs to place his finger on the right-angled equilateral prism 1. The control unit 23 controls each component of the apparatus for registering individualities.

In operation, an input candidate is instructed by the instruction unit 21 to place his finger on the right-angled equilateral prism 1. The instructions given by the instruction unit 21 may be displayed on a CRT with characters, given with audio signals, simultaneous use of both or in any other method.

The candidate places his finger on the right-angled equilateral prism 1 according to the instructions, specifies input timing by pressing an input switch (not shown) and inputs a finger image. An alternative arrangement is for the system itself to automatically verify finger placement by detecting changes in the density of the input image, whereby the finger image is automatically input.

The underside of the right-angled equilateral prism 1 is illuminated by the illuminating means 3. Since the finger pad is uneven, irrespective of the fingerprint, only the convex parts thereof come in contact with the right-angled equilateral prism 1 and light in these parts is not totally reflected but diffuses. Consequently, the light reaching the television camera 5 is reduced. On the other hand, the concave parts of the finger pad will not contact the prism, thus causing the light to be totally reflected, and the light is mostly received by the television camera 5. As a result, a fingerprint image having a contrast corresponding to the unevenness is received by the television camera 5.

The fingerprint image is input with, e.g., resolution of 16 pcs./mm and a size of 512 x 512 (pixels) and converted into a binary value in the processing unit 11. Subsequently, the whole fingerprint image is divided into 32 x 32 pieces of blocks in the same processing unit 11 and the representative direction within a fingerprint pattern existing in each block is quantized into eight directions in order that direction codes are extracted. The direction codes are stored in the distinctive feature storage unit 13-1 as those featuring the fingerprint.

Then a fingerprint image is input according to the instructions given by the instruction unit 21 and processed likewise, whereby the features thereof are stored in the second distinctive feature storage unit 13-2. In this manner, distinctive features of the fingerprint are successively stored in the third distinctive feature storage unit 13-3, the fourth distinctive feature storage unit 13-4 and the fifth distinctive feature storage unit 13-5. The distinctive features thus extracted from the input fingerprint five times are thus obtained through the aforementioned process.

A combination of any given two cases (i.e., 10 combinations in this case) is prepared from the five different kinds of features and then subjected to the identifying process to obtain functions of evaluation. A generally accepted practice is to obtain the difference between codes of positions corresponding to the direction codes extracted from the two fingerprint images to be compared first, the differences being squared and added up. In this case, the stronger the two fingerprints resemble, the smaller the value of the function of evaluation becomes. Equation (1) exemplifies the function of evaluation. The value of S represents a function of evaluation and $fp(i, j)$, $fq(i, j)$ designate the direction codes of Fp, Fq.

$$S_{p,q} = \Sigma \Sigma D_{i,j} \qquad (1)$$

where $D_{i,j} = d_{i,j}^2 \ (d_{i,j} \leq 4)$

$D_{i,j} = (8 - d_{i,j})^2 \ (d_{i,j} > 4)$

however, $d_{i,j} = |\ fp(i,j) - fq(i,j)\ |$

Now, there are direction codes extracted from five kinds of input fingerprint. If the combination of any given two cases is provided, the total number of combinations becomes 10. With respect to these ten combinations, the functions of evaluation expressed by Eq. (1) are considered.

Even if fingerprints are input with the greatest possible care, the ten kinds of functions of evaluation thus computed are assumed different because it would be impossible to input them equally. Given the five kinds of input fingerprints are F1, F2, F3, F4, F5 and, assuming the input fingerprints F4 and F5 are those obtained in poor input condition, the ten kinds of functions of evaluation may be divided into two groups from their sizes.

Group A (having small values)

S1, 2, S1, 3, S2, 3

Group B (having large values)

S1, 4, S1, 5, S2, 4, S2, 5, S3, 4, S3, 5, S4, 5

The utilization of these makes it possible to estimate the fingerprints obtained in poor input condition by combining two direction codes even when the fingerprints are obtained in poor input condition and simultaneously computing each value of the function of evaluation. In the case of the aforementioned example, F4 and F5 included not in the group A but in the group B are readily estimated as bad fingerprints.

Although F1, F2, F3 are estimated as good fingerprints conversely, the best one may be selected, provided one of them is to be selected, by selecting the combination giving the smallest value from among the three kinds of combinations in the group A first and what includes a component giving the smallest value between the two. Assuming the following size relations exist in the values of the group A:

S1, 2 > S2, 3 > S1, 3

In this case, F1 and F3 offer themselves as a candidate first and F3 giving a smaller value with respect to F2 is considered as the fingerprint obtained in the best input condition.

Although a reference has been made to a case where two out of five kinds are defective, a good fingerprint is readily estimated when the number of defectives is 1, 2 or 3. Incidentally, no combinations are included in the group A when the number of defectives becomes 4 or 5 and no good fingerprints cannot be estimated. In such a case, the operation of inputting data should be continued until what is included in the group A appears by giving instructions as to feeding inputs.

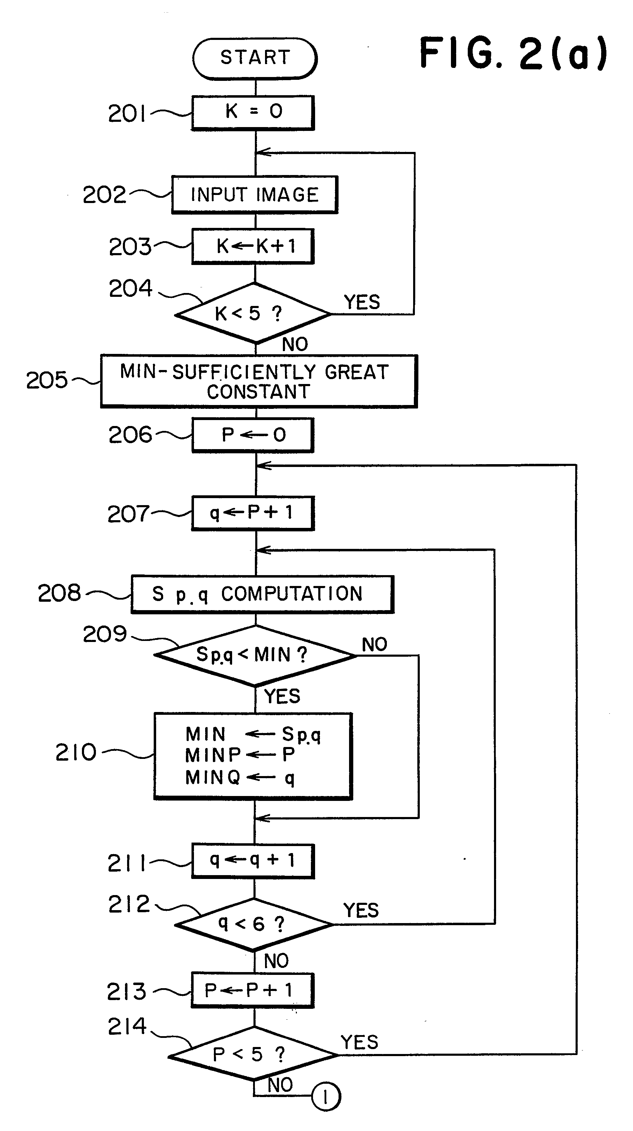

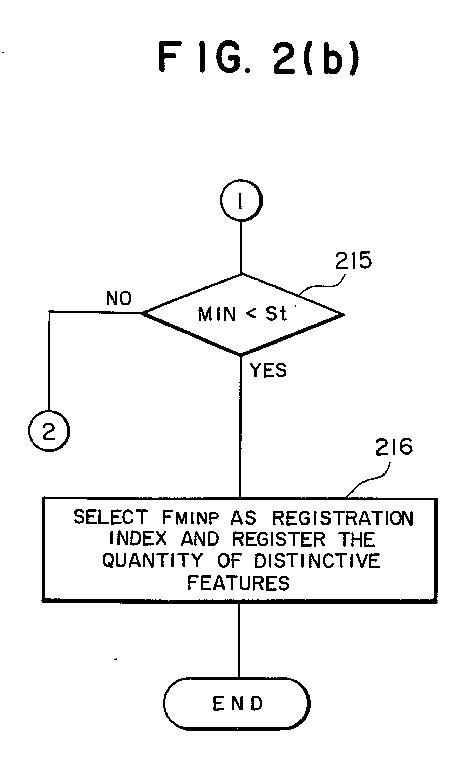

The theory of the present invention has been described in an understandable manner. Referring to Fig. 2, a description will subsequently be given of a simplified processing algorithm for selecting the best one from among the five kinds of fingerprints.

From among F1, F2, F3, F4, F5, a combination of two is selected to provide ten combinations and their functions of evaluation are totally computed to obtain the combination recording the smallest value. Provided it is the combination of F1 and F3 as in the case of the aforementioned example, F1 or F3 is selected as what is to be registered. With priority given to the effect of shortening the processing time, an example of selection of any one of F1 and F3 will be shown because whether F1 or F3 does not practically constitute a great difference.

The value of a parameter k is set at (0) (Step 201). An image is subsequently input from the read means 7 (Step 202), with the value of k incremented by one (Step 203), and it is determined if the value of k exceeds 5 (Step 204). In other words, the image at Step 202 is input five times.

A sufficiently great constant is set at a parameter MIN (Step 205) and (0) is set at a parameter p (Step 206), whereas a parameter q is set at p + 1 (Step 207), whereby S p,q is computed as mentioned above (Step 208).

Whether S p,q is smaller than the parameter MIN is determined (Step 209) and, if it is smaller, S p,q, p and q are set at MIN, MINP and MINQ, respectively (Step 210).

The value of q is subsequently incremented by one (Step 211) and whether the value of q exceeds 6 is determined (Step 212). If the value of q is not greater than 6, the procedure is returned to Step 208, whereas if q is not smaller than 6, the value of p is incremented by one (Step 213).

Whether the value of p exceeds 5 is subsequently determined (Step 214) and, if it is not greater than 5, the procedure is returned to Step 207, whereas if it is not smaller than 5, whether MIN is smaller than St is determined (Step 215). St is a preset threshold value.

If MIN is smaller than St, FMINP is selected as a registered fingerprint and its distinct features are registered (Step 216). Selection of either FMINP or FMINQ is optional.

In addition, F1- and F3-related functions of evaluation are separately computed so as to select optimum one of F1 and F3 by selecting what has a smaller value. In case the ten combinations of functions of evaluation initially computed are greater than the preset threshold value, the determination is given to the absence of a fingerprint to be registered or difficulty in making selection and inputting is demanded again.

As set forth above, according to the present invention, a candidate is input a plurality of times when physical features such as a fingerprint, etc. are registered and any given two cases are combined and then the combination is subjected to the same processing as what has been applied at the time of identification. The functions of evaluation are subsequently computed to estimate fingerprints obtained in poor input condition so that only the good fingerprint can be registered. This results in improvement of identifying accuracy.

In this embodiment, use can be made of fingerprints as physical features, the length between joints of fingers, the sum obtained by adding up pixel values in the direction perpendicular to the longitudinal direction of the finger, palm prints, voiceprints, retinal patterns, etc.

Fig. 4 is a block diagram illustrating the configuration of an apparatus for identifying an individual.

The apparatus for identifying an individual comprises a prismatic right-angled equilateral prism 1 for obtaining a finger image from the difference between the contact and noncontact of a finger 9 placed thereon in parallel to the axial direction therewith, an illuminating means 3 for irradiating the right-angled equilateral prism 1, a television camera 5 for converting the finger image thus obtained into an electrical signal, an A/D converter 25 for converting the electrical signal into digital quantity, an image memory 29 for storing the digital finger image obtained by the A/D converter 25, a control unit 23 forming a nucleus of processing such as identification, and a read/write unit 27 for writing the added-up signal of each person who is identified to an IC card 33 and reading data from the IC card 33. A host machine 31 controls switching of a door and sounding of an alarm buzzer in accordance with the signal from the control unit 23. What is controlled by the host machine 33 varies with the security system to which this apparatus for identifying an individual is applied.

A description will subsequently be given of a processing flow in the apparatus for identifying an individual. Identifying an individual is roughly classified into two processes: "registration" and "identification." The registration will be described first.

"Registration"

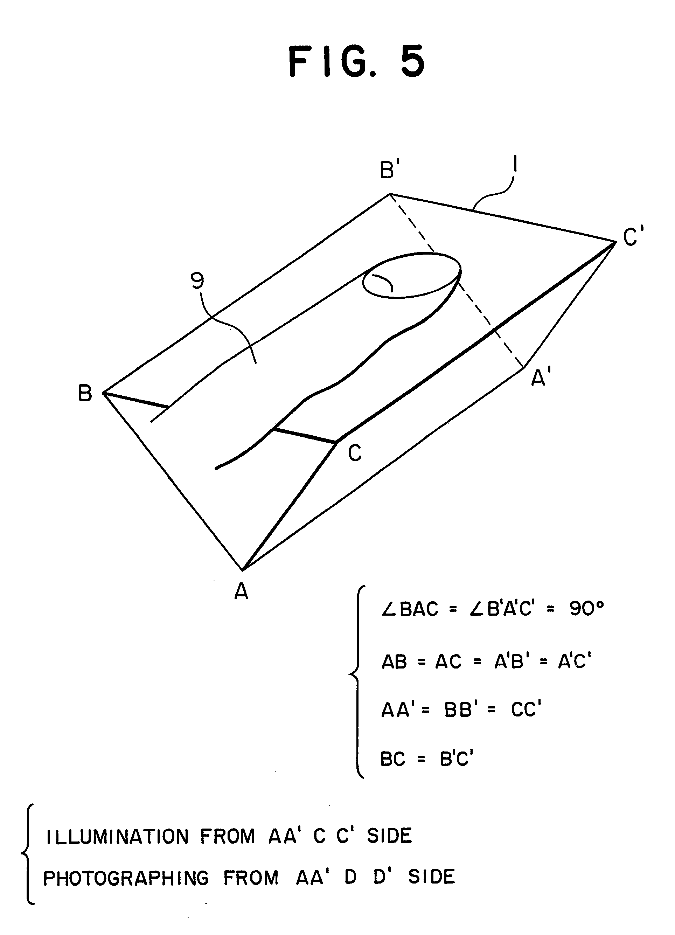

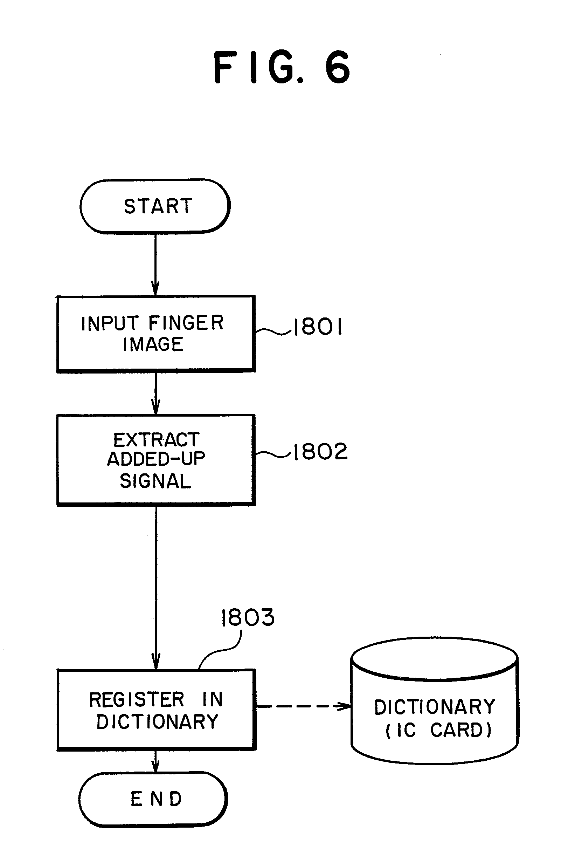

Fig. 6 shows a flowchart of registration. The finger is placed on the surface of the prism in such a manner as to satisfy the relation between the finger and the prism as shown in Fig. 5. The prism is illuminated from the $AA'$ $B'$ B side and photographed from the $AA'$ $C'$ C side to obtain a finger image (Step 1801). An added-up signal is extracted by adding up the densities of the finger image in the direction perpendicular to the longitudinal direction of the finger (Step 1802). Subsequently, the added-up signal is registered in a dictionary (IC card 33) (Step 1803).

"Identification"

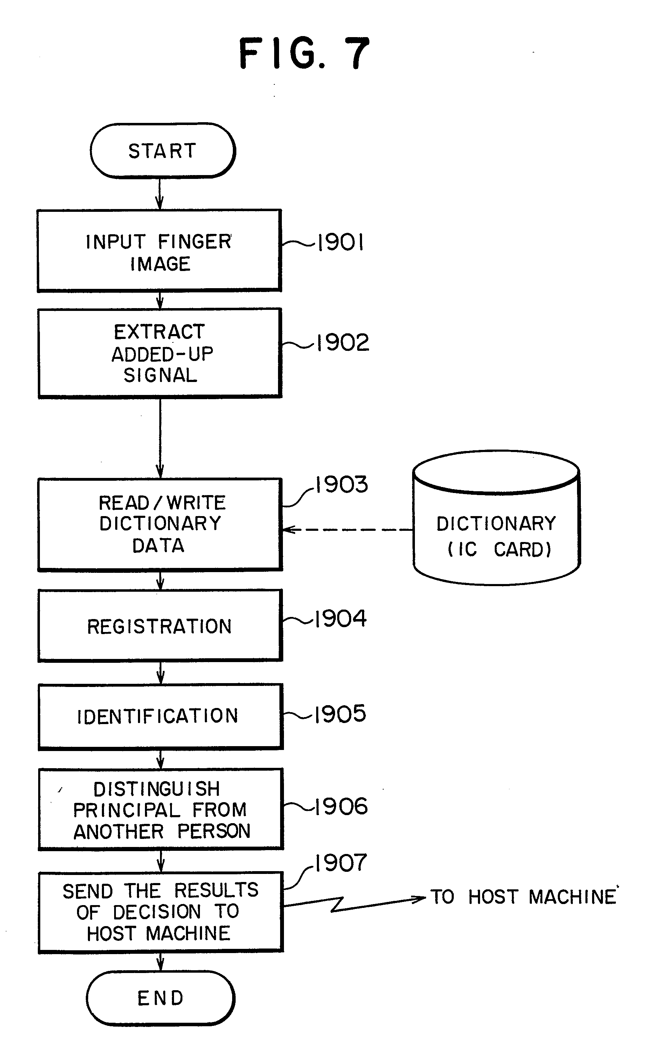

Fig. 7 shows a flowchart of identification. The inputting of the finger image and the extraction of the added-up signal in Steps 1091 and 1902 follow the same process as that in the case of registration. The added-up signal preregistered in the dictionary is read out (Step 1903) and the added-up signal read from the dictionary is put in correct alignment with what is obtained from the input image (Step 1904).

Given the added-up signal Ad(i) read from the dictionary, the added-up signal A(i) obtained from the finger image thus input, the number of factors N of each added-up signal, and added-up square errors S(m) of Ad(i) and A(i + m) shifted by m.
When $m \leq 0$,
$$S(m) = (1/(N - m)) \cdot \Sigma \{A(i + m) - Ad(i)\}^2 \qquad (2)$$
When $m < 0$,
$$S(m) = (1/(N + m)) \cdot \Sigma \{A(i + m) - Ad(i)\}^2 \qquad (3)$$
S(m) represents a parameter indicating consistency of A(i+m) and Ad(i), i.e., the smaller the value of S(m), the greater the consistency. The correct alignment is deemed complete at the position of M where the value of S(m) is minimized while making m change within a predetermined range; the then value of S(M) is made the result of identification (Step 1905).

In the same manner as aforementioned, the principal and another person is distinguished (Step 1906). The resulting decision is sent to the host machine 31 (Step 1907), where processing is conducted in accordance with the results of decision.

Although the prism is illuminated from the $AA'$ $B'$ B side in Fig. 5, it may be illuminated from the $AA'$ $C'$ C side used for photographing.

As set forth above, according to the present invention, not only more accurate identification but also improvement in identifying accuracy becomes possible because there develops almost no difference between the actual length in each portion of a finger and the length on the pickup image.

In this embodiment, moreover, use can be made of the added-up signal obtained by adding up the densities of the finger image in the direction perpendicular to the longitudinal direction of the finger, the length of the portion partitioned with the joint of the finger, the length of the finger, etc.

## Claims

(1) An apparatus for registering individualities, comprising:
input means for inputting the same data representing physical features of an individual a plurality of times;
comparison means for comparing the data representing said physical features input by said input means, and
selection means for selecting the data to be registered from among the data thus input on the basis of the results of comparison made by said comparison means.

(2) The apparatus for registering individualities according to claim 1, wherein said selection means includes means for obtaining highest similarity from among data input by said input means on the basis of the results of comparison made by said comparison means, and
said apparatus further comprises means for increasing the number of inputs repeated by said input means when the similarity is lower than a specified value while the similarity of the data selected by said selection means is compared with the specified value.

(3) The apparatus for registering individualities according to claim 1, wherein said comparison means includes means for obtaining the similarity between pickup images by comparing the images thus picked up, and
said selection means includes means for selecting highest similarity from among the images picked up by said input means on the basis of the similarity computed.

(4) An apparatus for registering individualities, comprising:
read means for reading physical feature of an individual;
storage means for storing signals representing physical features of the individual read by said read means a plurality of times, respectively;
comparison means for comparing the signals stored in said storage means;
selection means for selecting a signal to be registered from among those stored in said storage means according to the results of comparison made by said comparison means; and
registration means for registering the signal selected by said selection means.

(5) The apparatus for registering individualities according to claim 4, wherein said physical features are fingerprints.

(6) The apparatus for registering individualities according to claim 4, wherein said physical features are lengths between joints of finger.

(7) The apparatus for registering individualities according to claim 4, wherein said physical features are quantities obtained by adding up pixel values in the direction perpendicular to the longitudinal direction of finger.

(8) An apparatus for identifying an individual, comprising:
a light source;
a prismatic prism on which a finger is placed in parallel to the axial direction;
read means having pickup means for picking up the light emitted from said light source and reflected from said finger;
storage means for storing an output signal of said read means; and
identifying means for identifying the signal read by said read means with the signal stored in said storage means at the time of identification.

(9) The apparatus for identifying an individual according to claim 8, wherein said identifying means includes means for obtaining an added-up signal by adding up the densities of a finger image in the direction perpendicular to the longitudinal direction of the finger for identifying purposes.

(10) The apparatus for identifying an individual according to claim 8, wherein said identifying means includes means for measuring a length of the portion partitioned with the joints of a finger.

(11) The apparatus for identifying an individual according to claim 8, wherein said identifying means including means for measuring a length of a finger.

FIG. 1

# FIG. 2(a)

```
                    ┌─────────────┐
                    │    START    │
                    └─────────────┘
                           │
201 ───            ┌─────────────┐
                   │   K = 0     │
                   └─────────────┘
                           │
                           │◄──────────────────────┐
202 ───            ┌─────────────┐                  │
                   │ INPUT IMAGE │                  │
                   └─────────────┘                  │
                           │                        │
203 ───            ┌─────────────┐                  │
                   │  K ← K + 1  │                  │
                   └─────────────┘                  │
                           │                        │
204 ───              ╱─────────╲         YES        │
                    ╱  K < 5 ?  ╲──────────────────┘
                    ╲           ╱
                     ╲─────────╱
                          │ NO
205 ───    ┌────────────────────────────┐
           │ MIN-SUFFICIENTLY GREAT     │
           │        CONSTANT            │
           └────────────────────────────┘
                           │
206 ───            ┌─────────────┐
                   │   P ← 0     │
                   └─────────────┘
                           │
                           │◄──────────────────────────────┐
207 ───            ┌─────────────┐                          │
                   │  q ← P + 1  │                          │
                   └─────────────┘                          │
                           │                                │
                           │◄──────────────────┐            │
208 ───    ┌────────────────────────────┐       │            │
           │   S p.q  COMPUTATION       │       │            │
           └────────────────────────────┘       │            │
                           │                     │            │
209 ───              ╱─────────╲        NO        │            │
                    ╱ Sp.q < MIN?╲──────────────┐ │            │
                    ╲           ╱               │ │            │
                     ╲─────────╱                │ │            │
                          │ YES                 │ │            │
210 ───    ┌────────────────────────────┐       │ │            │
           │   MIN  ← S p.q             │       │ │            │
           │   MINP ← P                 │       │ │            │
           │   MINQ ← q                 │       │ │            │
           └────────────────────────────┘       │ │            │
                           │◄───────────────────┘ │            │
211 ───            ┌─────────────┐                 │            │
                   │  q ← q + 1  │                 │            │
                   └─────────────┘                 │            │
                           │                       │            │
212 ───              ╱─────────╲         YES        │            │
                    ╱  q < 6 ?  ╲───────────────────┘            │
                    ╲           ╱                                │
                     ╲─────────╱                                 │
                          │ NO                                   │
213 ───            ┌─────────────┐                               │
                   │  P ← P + 1  │                               │
                   └─────────────┘                               │
                           │                                     │
214 ───              ╱─────────╲         YES                      │
                    ╱  P < 5 ?  ╲────────────────────────────────┘
                    ╲           ╱
                     ╲─────────╱
                          │ NO
                          ○ 1
```

# FIG. 2(b)

```
                    ( I )
                      |
                      |
         NO        ╱‾‾‾‾‾╲        215
      ┌──────────<  MIN < St  >
      │            ╲____╱
      │               |
     ( 2 )          YES
                      |
                      |              216
         ┌────────────────────────────┐
         │  SELECT FMINP AS REGISTRATION │
         │   INDEX AND REGISTER THE      │
         │  QUANTITY OF DISTINCTIVE      │
         │         FEATURES              │
         └────────────────────────────┘
                      |
                      |
                  (  END  )
```

# FIG. 3

# FIG. 4

# FIG. 5

$\angle BAC = \angle B'A'C' = 90°$

$AB = AC = A'B' = A'C'$

$AA' = BB' = CC'$

$BC = B'C'$

ILLUMINATION FROM AA' C C' SIDE

PHOTOGRAPHING FROM AA' D D' SIDE

# FIG. 6

```
          ┌──────────┐
          │  START   │
          └──────────┘
                │
                ▼
      ┌─────────────────┐
      │  INPUT FINGER   │────  1801
      │     IMAGE       │
      └─────────────────┘
                │
                ▼
      ┌─────────────────┐
      │    EXTRACT      │
      │   ADDED-UP      │────  1802
      │    SIGNAL       │
      └─────────────────┘
                │
                │        1803
                │       ⌐
                ▼
      ┌─────────────────┐          ┌──────────────┐
      │  REGISTER  IN   │ ──  ──▶  │  DICTIONARY  │
      │   DICTIONARY    │          │  (IC CARD)   │
      └─────────────────┘          └──────────────┘
                │
                ▼
          ┌──────────┐
          │   END    │
          └──────────┘
```

# F I G. 7

```
        ( START )
            |
            v
   +------------------+
   |   INPUT FINGER   |~1901
   |      IMAGE       |
   +------------------+
            |
            v
   +------------------+
   |     EXTRACT      |~1902
   |    ADDED-UP      |
   |     SIGNAL       |
   +------------------+
            |
            v
   +------------------+
   |   READ/WRITE     |~1903        DICTIONARY
   |   DICTIONARY     | <--------    (IC CARD)
   |     DATA         |
   +------------------+
            |
            v
   +------------------+
   |  REGISTRATION    |~1904
   +------------------+
            |
            v
   +------------------+
   | IDENTIFICATION   |~1905
   +------------------+
            |
            v
   +------------------+
   |   DISTINGUISH    |~1906
   | PRINCIPAL FROM   |
   | ANOTHER PERSON   |
   +------------------+
            |           1907
            v
   +------------------+
   | SEND THE RESULTS |
   |  OF DECISION TO  | ------> TO HOST MACHINE
   |   HOST MACHINE   |
   +------------------+
            |
            v
        ( END )
```